(19) Europäisches Patentamt
European Patent Office
Office européen des brevets

(11) **EP 0 684 944 B1**

(12) # FASCICULE DE BREVET EUROPEEN

(45) Date de publication et mention de la délivrance du brevet:
**25.07.2001 Bulletin 2001/30**

(21) Numéro de dépôt: **94907600.4**

(22) Date de dépôt: **21.02.1994**

(51) Int Cl.$^7$: **C07D 233/54**, A61K 31/415, A61K 7/48

(86) Numéro de dépôt international:
**PCT/FR94/00189**

(87) Numéro de publication internationale:
**WO 94/19325 (01.09.1994 Gazette 1994/20)**

(54) **PRODUIT DE COUPLAGE DE L'HISTAMINE ET D'UN ACIDE AMINE**

KUPPLUNGSPRODUKT ZWISCHEN EINER AMINOSAEURE UND HISTAMIN

COUPLING PRODUCT OBTAINED FROM HYSTAMINE AND AN AMINO ACID

(84) Etats contractants désignés:
**BE CH DE DK ES FR GB GR IE IT LI MC NL SE**

(30) Priorité: **22.02.1993 FR 9302295**
**30.08.1993 FR 9310486**

(43) Date de publication de la demande:
**06.12.1995 Bulletin 1995/49**

(73) Titulaires:
- **EXSYMOL S.A.M.**
**MC-98000 Monte Carlo (MC)**
- **BABIZHAYEV, Marc**
**74, Moscou, 127434 (RU)**

(72) Inventeurs:
- **BABIZHAYEV, Marc**
**Moscou, 127434 (RU)**
- **SEGUIN, Marie-Christine**
**F-06360 Eze (FR)**

(74) Mandataire: **Nevant, Marc et al**
**Cabinet Beau de Loménie,**
**158, rue de l'Université**
**75340 Paris Cedex 07 (FR)**

(56) Documents cités:
**WO-A-92/09298** **WO-A-93/04690**

- **CHEMICAL ABSTRACTS, vol. 111, no. 19, 1989, Columbus, Ohio, US; abstract no. 167340h, M. A. BABIZHAYEV 'Antioxidant activity of L-carnosine, a natural histidine-containing dipeptide in crystalline form' page 74 ; & BIOCHIM. BIOPHYS. ACTA vol. 1004, no. 3 , 1989 pages 363 - 371**
- **BULLETIN OF THE CHEMICAL SOCIETY OF JAPAN vol. 54 , 1981 , TOKYO JP pages 1101 - 1104 M. NANASAWA ET AL. 'Models for visual pigments. The effect of the imidazolyl group on the absorption maxima of the retinal Schiff base'**
- **N.V. GULYAEVA, : "Prospects for the desing of carnosine-based drugs: some new principles", BIOKHIMIYA, (ENGLISH TRANSLATION), Septembre 1992, Vol. 57, no. 9, pages 1398 à 1403**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 684 944 B1

## Description

La présente invention concerne des produits à base d'histamine et plus particulièrement un produit de couplage entre l'histamine ou l'histamine méthyl-substituée et un acide aminé, son procédé de préparation et ses applications en tant que principe actif sur le plan thérapeutique et cosmétologique.

Les propriétés biologiques des dipeptides tels que la β-alanyl-histidine sont particulièrement intéressantes dans la mesure où elles contribuent à renforcer les moyens naturels de défense et de réparation de l'organisme. Toutefois, les composés comportant un amino-acide ou plus dans leur structure, tels que la β-alanyl-histidine, forment une classe de principes actifs généralement bien tolérés par l'organisme mais dont l'efficacité est considérablement réduite par le fait que ces produits sont rapidement dégradés par l'organisme. De plus, bien que la sensibilité aux désactivations enzymatiques soit considérablement réduite pour les très petits peptides (J. Dressman "Opportunities for peptide absorbtion in the GI tract", Communication GTRV 1992, Paris), la désactivation enzymatique des dipeptides pourrait conduire dans certains cas, à la libération d'histamine. Cette activité "pro-histaminique", associée à la perte d'activité du dipeptide d'origine, n'est pas souhaitable dans le cadre de cette invention.

La demande internationale WO 93/04690, déposée le 9 septembre 1992 et publiée le 18 mars 1993 concerne une méthode pour le traitement des complications et de la pathologie du diabète à l'aide de différents composés dont la carcinine.

La demande internationale WO 92/09298 concerne une méthode pour retarder, prévenir et/ou inverser la sénescence des cellules à l'aide d'une composition comprenant au moins 10 mM d'un principe actif ayant des propriétés chimiques similaires à celles de la carnosine.

Le but principal de l'invention est de mettre au point des produits peptoïdes proches des dipeptides mentionnés ci-dessus, mais dont l'efficacité n'est pas réduite du fait qu'ils ne sont pas dégradés par l'organisme.

Un autre but de l'invention est de réaliser un produit peptoïde à base d'histamine ayant des propriétés qui renforcent les moyens naturels de défense et de réparation de l'organisme.

Encore un autre but de l'invention est de réaliser un produit peptoïde tel que défini ci-dessus, et dont la biodisponibilité est améliorée en acétylant l'extrémité aminée.

Encore un autre but de l'invention est de réaliser un produit peptoide ayant un groupement acétylé tel que défini ci-dessus, de sorte que l'hydrolyse par des enzymes du type acétylpeptide hydrolase permette la libération in situ d'un produit actif par effet retard.

L'objet principal de l'invention est donc un produit pseudo-dipeptide obtenu par couplage entre l'histamine ou l'histamine méthyl-substituée et un acide aminé ayant pour formule :

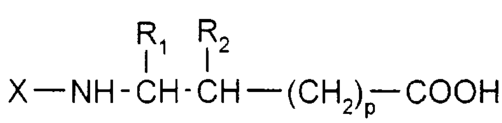

dans laquelle :

- X représente un atome d'hydrogène ou un groupe acétyle ;
- $R_1$ représente un atome d'hydrogène ou un groupe phényle ;
- $R_2$ représente un atome d'hydrogène ou un groupe méthyle ;
- p est un nombre entier compris entre 0 et 3 inclus ;

la liaison covalente avec l'histamine ou l'histamine méthyl-substituée étant une liaison peptidique entre le groupe carboxylique de l'acide aminé et le groupe amine de l'histamine ;
ledit produit étant différent de la β-alanyl-histamine ou de la 6-aminocaproyl-histamine.

Parmi les acides aminés répondant à la formule de l'invention, les acides aminés suivants donnent les meilleurs résultats :

la β-alanine de formule

$$H_2N - CH_2 - CH_2 - COOH$$

l'acide γ-aminobutyrique de formule

$$H_2N - CH_2 - CH_2 - CH_2 - COOH$$

l'acide β-aminoisobutyrique de formule

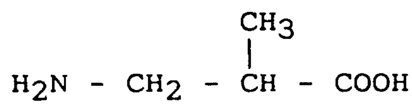

l'acide 5-aminovalérique de formule

$$H_2N - CH_2 - CH_2 - CH_2 - CH_2 - COOH$$

l'acide 3-phényl-3-aminopropionique

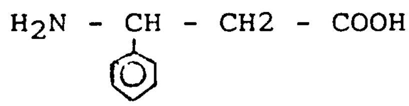

l'acide 6-aminocaproïque

$$H_2N - (CH_2)_5 - COOH$$

l'acide 4-méthyl-aminobutyrique

$$CH_3 - NH - CH_2 - CH_2 - CH_2 - COOH$$

l'acide DL-β-aminobutyrique

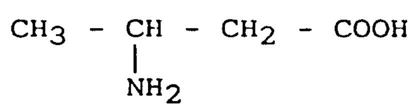

l'acide L-glutamique

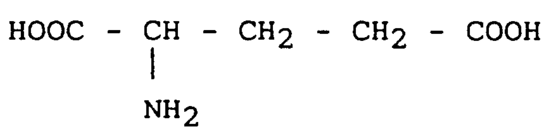

les acides aminés suivants dans lesquels l'extrémité aminée a été acétylée. En effet, l'hydrolyse de ce groupement par des enzymes (du type acétylpeptide hydrolase) permet la libération in situ d'un produit actif par effet retard.

l'acide N-acétyl-β-alanine

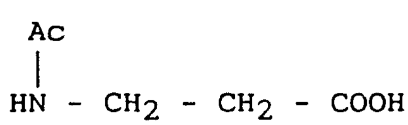

l'acide N-acétyl-3-phényl-3-aminopropionique

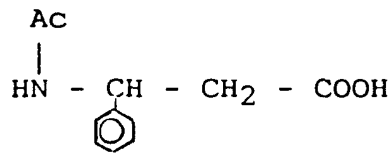

Toutefois, la β-alanine est l'acide aminé qui permet d'obtenir un produit pseudo-dipeptide réalisant parfaitement les buts de l'invention. La β-alanine peut être couplée soit à l'histamine de formule

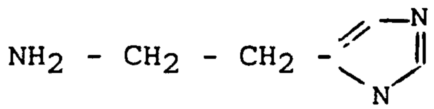

soit à la 1-méthyl-histamine ou 1-méthyl-imidoazoléthylamine

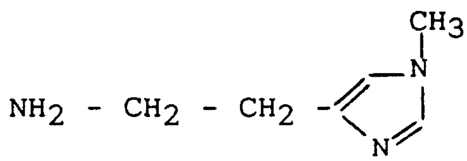

soit à la 3-méthyl-histamine ou 3-méthyl-imidoazoléthylamine

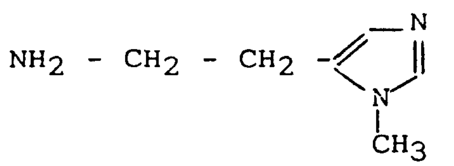

Le produit pseudo-dipeptide préféré dans le cadre de l'invention est la β-alanyl-histamine de formule

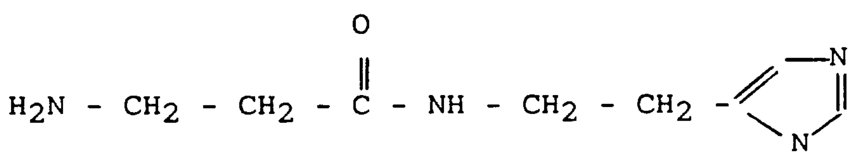

La préparation du produit pseudo-dipeptide selon l'invention peut se faire, soit en utilisant un procédé chimique, soit en utilisant une méthode de synthèse en tout ou en partie enzymatique.
Le procédé de préparation chimique se présente selon le shéma suivant:

$$AA + X + Y \rightarrow X\text{-}AA\text{-}Y \qquad (1)$$

$$X\text{-}AA\text{-}Y + Hist\ (.2Hcl) \rightarrow X\text{-}AA\text{-}Hist \qquad (2)$$

$$X\text{-}AA\text{-}Hist \rightarrow AA\text{-}Hist \qquad (3)$$

La première étape du procédé consiste à rendre l'amino-acide (AA) N-protégé par un groupement X, et O-activé par un groupement Y.

La N-protection est effectuée de préférence par le remplacement d'un atome d'hydrogène dans l'amine de l'acide aminé par le groupement X qui peut être un radical acyl, acyloxy,... Parmi les groupements protecteurs les plus intéressants on peut citer le benzyl-oxycarbonyle, le ter-butyloxy-carbonyle (BOC), le 9-fluorényl-méthyl-oxycarbonyle (FMOC), les radicaux benzyle, phthaloyle, 2-nitrophényl-sulfényle, le trifluoro-acétyle, le ter-butyloxy-carbonyle étant préféré.

Bien qu'il soit possible de s'en dispenser, l'activation O est une des caractéristiques du procédé d'obtention du produit pseudo-dipeptide selon l'invention. Cette activation réalisée de préférence par estérification de la fonction carboxylique de l'acide aminé par un composé choisi dans le groupe consistant en: alcool de cyanométhyle, o-nitrophénol, 2,4,5-trichlorophénol, p-nitrophénol, 2,4-dinitrophénol, pentachlorophénol, pentafluorophénol, N-hydroxyphtalimide, N-hydroxysuccinimide, 1-hydroxypipéridine et 5-chloro-8-hydroxy-quinoline.

Ainsi, si on utilise le pentafluorophénol en tant que groupement Y, la réaction s'écrit, si R - COOH est l'acide aminé selon l'invention

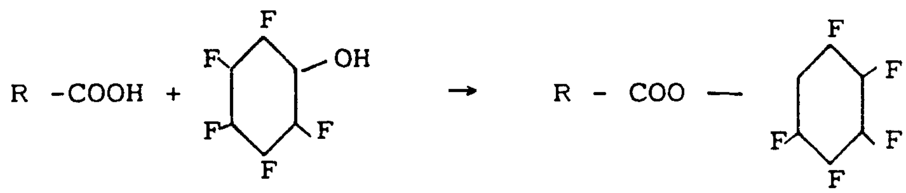

La deuxième étape du procédé de préparation est le couplage avec l'histamine qui peut se faire avec ou sans agent de couplage, en faisant réagir l'acide aminé N-protégé et O-activé avec l'histamine de préférence sous forme de dichlorhydrate. I1 faut noter que l'agent de couplage n'est pas indispensable avec un acide aminé O-activé.

Le couplage sans agent de couplage s'effectue dans un solvant organique (par exemple chloroforme,1,2-dimethoxyethane, diméthylformamide... ) en présence d'acide (par exemple acide acétique...) ou de base (par exemple triétylamine...) dans un solvant hydro-organique (par exemple eau-pyridine ou eau-1,2-diméthoxyethane...) en présence de base (par exemple soude ou bicarbonate de sodium...) puis d'acide (par exemple acide chlorhydrique...); dans des conditions catalytiques (par exemple imidazole, N-ethylmorpholine...)...

Si un agent de couplage est utilisé, cet agent peut-être par exemple le dicyclohexyl-carbodiimide, le 1-isobutyloxycarbonyl-2-isobutyloxy-1,2-dihydroquinoline, le carbonyldiimidazole, Woodward Reagent K, α-chlorovinyl ethyl ether, α,α-dichlorodiethyl Ether, dichloromethyl methyl ether, DCC et additifs, DCC-pentachlorophénol, DCC-pentafluorophénol, cyanamide, cetenimines et cétènes, sels d'oxazolinium, EEDQ ('1-ethoxycarbonyl-2-ethoxy-1,2-dihydroquinoléine), ynamines acylphosphoniums, triphénylphosphite et imidazole, complexes de cuivre II, SiC14...

Dans la troisième étape, le groupement X ou N-protecteur est éliminé. Cette élimination se fait, selon les groupements protecteurs, par hydrogénolyse, par réduction par le sodium dans l'ammoniac liquide, par hydrazinolyse, par acidolyse, par hydrolyse ou par voie enzymatique. La solution préférée consiste à effectuer cette étape de déprotection par acidolyse par l'acide chlorhydrique dans l'acide acétique. Dans ce dernier cas, le produit pseudo-dipeptide est obtenu sous forme de chlorhydrate, et il faut le traiter par des résines pour recueillir le produit base.

A titre d'exemple, le procédé de préparation de la β-alanyl-histamine peut se faire de la façon suivante:

370 mg de chlorhydrate d'histamine (2 mmol) sont dissous dans 7 ml de diméthylformamide (DMF). 0.56 ml de triéthylamine (4 mmol) et 800 mg de N-terbutyloxycarbonyl-β-alanyl-pentafluorophényl-ester (2.25 mmol) sont ajoutés au mélange. Le mélange est agité 30 minutes à 0°C puis 3 heures à température ambiante. Le résidu est filtré puis lavé avec du diméthylformamide. Le diméthylformamide est évaporé sous vide. De l'éther est ajouté au résidu et le composé huileux N-terbutyloxycarbonyl-β-alanyl-histamine est recueilli par décantation. Le chlorhydrate de β-alanyl-histamine est ensuite obtenu par traitement du N-terbutyloxycarbonyl-β-alanyl-histamine par de l'acide chlorhydrique en solution dans l'acide acétique pendant 30 minutes. L'acide acétique est partiellement évaporé sous vide et de l'éther est rajouté au résidu. Le résidu est alors filtré. La recristallisation par le système éthanol/méthanol (50/50) - éther éthylique conduit à l'obtention de 300 mg de chlorhydrate de β-alanyl-histamine. Enfin, la β-alanyl-histamine base est obtenue par passage du chlorhydrate correspondant sur des résines.

La méthode de synthèse enzymatique se fait en utilisant la réaction de couplage suivante, une fois que l'amino-acide (AA) est N-protégé (groupement X) et O-activé (groupement Y):

$$X\text{-}AA\text{-}Y + hist \xrightarrow{enzyme} X\text{-}AA\text{-}hist + Y$$

Dans le cas présent, la N-protection dont la préparation a déjà été décrite dans le procédé de préparation chimique, permet aussi d'accroître la solubilité de l'acide aminé dans le milieu réactionnel. Ceci peut diriger le choix du groupement X de façon à augmenter la solubilité de l'acide aminé dans les milieux organiques. Ainsi, X est de préférence le radical benzyl-oxycarbonyle

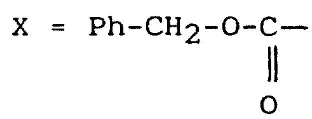

L'activation O peut se faire par estérification de la fonction carboxylique par un alcool choisi dans le groupe consistant en: alcools aliphatiques, de préférence l'éthanol, les halogéno-alkylalcools tels que le 2,2,2-trichloro-éthanol, les alcools aromatiques tels que le phénol, ainsi que les alcools déjà cités pour le procédé de préparation chimique. Il faut toutefois exclure les alcools tertiaires.

La réaction de couplage avec l'histamine base (ou ses dérivés méthylés) ou un sel d'histamine (ou ses dérivés méthylés), par exemple le dichlorhydrate d'histamine, est réalisée dans une variété de solvants organiques tels que les solvants hydrocarbonés aliphatiques (cyclohexane, heptane...) ou aromatiques (toluène), les alcools tertiaires (t-butanol, ter-amylalcool), les halogénures d'alkyle (dichlorométhane), les éthers (isopropyléther), l'acétonitrile, le diméthylformamide ou le diméthylsulfoxyde. Ces solvants peuvent être utilisés seuls ou en association, anhydres ou en présence d'une faible quantité d'eau.

La réaction peut être conduite en présence ou non d'une base, telle que la triéthylamine.

Le catalyseur enzymatique est une hydrolase (lipase, protéase) d'origine microbienne ou animale ou végétale. Il peut être sous une forme pure ou non purifiée.

On peut ainsi prendre comme catalyseur les lipases extraites de micro-organismes: Pseudomonas sp., Candida Rugosa, Mucor, ou d'origine animale: lipase pancréatique du porc (LPP), des protéases: trypsine, chimotrypsine, substilisine, papaine...

Le catalyseur, non soluble dans le milieu réactionnel, est dispersé dans le solvant seul ou immobilisé sur un support inerte afin de faciliter son recyclage.

La réaction est effectuée à une température comprise entre 4°C et 70°C, mais de préférence entre 35°C et 45°C sous agitation.

Le produit de couplage est recueilli par filtration ou après extraction avec un solvant approprié. la déprotection et la purification peuvent être réalisées selon la méthode décrite en relation avec le procédé de préparation chimique. Toutefois, cette étape de déprotection peut être réalisée par une réaction enzymatique selon le schéma:

$$X\text{-}AA\text{-}hist \xrightarrow[R\text{-}OH]{enzyme} AA\text{-}hist + X\text{-}OR$$

où R = H ou un radical alkyle.

Le protocole est similaire à celui décrit pour l'étape de couplage. Toutefois, dans le cas où R = H la réaction est conduite dans l'eau.

Pour l'étape de couplage, il peut être envisagé des solutions moins favorables, mais permettant de réduire les coûts de production de façon importante, en utilisant soit un amino-acide non N-protégé (où X = H), mais O-activé, soit un amino-acide N-protégé, mais non O-activé (où Y = H), soi même l'amino-acide de départ (X = Y = H). Les conditions opératoires sont similaires à celles décrites précédemment pour le cas où l'amino-acide est N-protégé et O-activé.

Dans l'exemple suivant, la N-acétyl-β-alanyl-histamine est préparée comme suit:

- 2,38 g (15 mmoles) de N-acétyl--alanine éthyl ester et 1,11 g (10 mmoles) d'histamine sont dissous dans 40 ml d'un mélange tert-amylalcool-heptane (25:75).

- 0,5 g de lipase Amano P (Pseudomonas sp.) sont ajoutés et la suspension est chauffée à 40°C, sous agitation pendant 48 heures.

La disparition complète d'histamine est vérifiée par HPLC (R.P.C-18-H20-CH3CN-TFA:99/1/0,1).
Le catalyseur est alors récupéré par filtration. Le solvant organique est évaporé sous vide pour donner un composé huileux. Ce résidu est trituré dans l'éther éthylique afin d'éliminer l'excès de N-acétyl-β-alanine éthyl ester. On obtient 1,9 g de N-acétyl-β-alanyl-histamine.

**Propriétés pharmacologiques**

Comme il a déjà été mentionné, les produits pseudo-dipeptides selon l'invention, peuvent potentialiser certains mécanismes de défense et de réparation de l'organisme. Ils s'opposent ainsi au vieillissement et à la dégénérescence des tissus (oculaires, musculaires, cutanés,...) et ils favorisent la cicatrisation.
Les propriétés anti-oxydantes de ces produits, et plus précisément leur caractère de piégeurs de radicaux libres, et leur capacité à éliminer les produits de péroxydation toxiques pour l'organisme, rendent compte, en partie, des propriétés pharmacologiques observées. La capacité de ces produits à s'opposer au "stress oxydatif", et donc à suppléer aux systèmes de défense anti-oxydative de l'organisme, leur permet d'intervenir dans diverses pathologies. On peut citer leur action anti-inflammatoire, leur rôle de radioprotecteur lors de traitements en radiothérapie, leurs propriétés anti-athérosclérose (inhibition de l'oxydation des LDL), anti-cataracte ou anti-tumorales qui sont maintenant bien documentées.
Comme il a déjà été mentionné, les produits pseudo-dipeptides selon l'invention présentent des propriétés d'antivieillissement et d'antidégénérescence des tissus cutanés et muqueuses. Ces propriétés sont dues de façon générale à l'activité antioxydante de ces produits, et en particulier aux activités anti-radicaux libres, pseudo-péroxydase, antiréticulation, et de régénération tissulaire qu'ils démontrent.
De plus, de multiples arguments expérimentaux, analytiques et épidémiologiques, militent en faveur de la théorie selon laquelle l'accumulation des dommages biochimiques provoqués par les R.L. constituerait le processus essentiel du vieillissement. Il est notamment clair que l'exposition au rayonnement solaire, responsable de la formation d'espèces radicalaires dérivées de l'oxygène, est une cause du vieillissement cutané prématuré, comme elle est le facteur principal dans le formation de la cataracte dite "sénile".
La plupart des constituants cellulaires représentent en effet des cibles potentielles pour l'attaque des radicaux libres. Les acides gras insaturés entrant dans la composition de phospholipides membranaires sont particulièrement sensibles à leur agression. Leur oxydation conduit à une désorganisation membranaire, à la perte des composants intracellulaires, à la formation d'aldéhydes (toxiques) et de complexes lipoprotéiques (lipofuschine). Les protéines sont également des cibles pour les radicaux libres, et leur attaque conduit à la dénaturation des protéines, à leur coupure. L'altération du tissu conjonctif par les RL est un élément important de leur action déstructurante. Les constituants glucidiques sont également attaqués, l'acide hyaluronique est dépolymérisé, les récepteurs membranaires glycoprotéiques sont altérés. Enfin, les acides nucléiques sont des cibles d'une grande importance fonctionnelle.
Une autre caractéristique des produits pseudo-dipeptides selon l'invention rendant compte de la protection des protéines vis-à-vis du "stress oxydatif", est la présence du groupement imidazole de l'histamine. En effet, les phénomènes oxydatifs, responsables de la dégradation des protéines, semblent impliquer tout particulièrement le cycle imidazole de l'amino-acide histidine (Ref."Hydroxyl radical mediated damage to proteins with special reference to the cristallins" - Biochemistry, vol.31 (1992), p.4296). Il apparait ainsi que ces composés peuvent jouer un rôle de "leurre" pour les R.L.
Les produits pseudo-dipeptides selon l'invention peuvent s'oppposer au "stress oxydatif" à un autre niveau. Ces composés sont capables de réagir, non plus sur l'espèce radicalaire, mais en aval du phénomène oxydatif, sur des sous-produits toxiques, les péroxydes, issus de la réaction des R.L. sur certains constituants cellulaires. Cette activité dite "péroxydase", par analogie avec le nom donné aux enzymes chargées dans l'organisme d'éliminer les péroxydes, permet de neutraliser ces péroxydes et ainsi d'empêcher des réactions en chaine, qui conduisent notamment à la rupture des membranes cellulaires. On obtient ainsi une activité réparatrice.
Plusieurs tests in vitro ont été réalisés afin de mettre en évidence les différentes propriétés antioxydantes évoquées.
PROTOCOLE décrit par J.M.C. Gutteridge dans Biochemistry Journal, vol 224 (1984), p. 761-767:

- substrat d'oxydation: désoxyribose,
- système de production d'anions superoxydes: xanthine oxydase/hypoxanthine,
- détection: acide thiobarbiturique/malondialdéhyde (MDA)

| | % inhibition |
|---|---|
| Témoin | 0 |
| L-carnosine (10mM) | 37 |
| β-alanyl-histamine(10mM) | 49 |

Cette activité s'exerce également pour la protection des phospholipides membranaires. Les radicaux libres oxygénés réagissent avec les phospholipides pour former des produits instables qui, en se dégradant, entraînent la rupture de la membrane cellulaire.

Les tests suivants illustrent cette action.

PROTOCOLE 1

- substrat d'oxydation: liposomes de phosphatidylcholine
- système de production de radicaux libres: Fe/acide ascorbique,
- détection: acide thiobarbiturique (TBA)/ malondialdéhyde (MDA)

Protocole décrit dans "Anti-oxydant activity of L-carnosine, a natural histidine-containing dipeptide in cristallin lens". Biochem. Biophys. Acta (1989), vol 1004, p.363-371.

| | ηmol MDA mg phospholipides | % d'inhibition |
|---|---|---|
| Témoin(sans inhibiteur) | 4,4 | 0 |
| β-alanyl-histamine(10mM) | 2,5 | 42 |
| Carnosine(10mM) | 2,0 | 53 |
| Histamine(10mM) | 6,8 | -63 |
| Histamine 1μM | 6,4 | -45 |
| Histidine 1mM | 6,6 | -48 |

PROTOCOLE 2:

- substrat d'oxydation: acide linoléique (0,25 mg/ml),
- système de production de radicaux libres: Fe/acide ascorbique,
- détection: acide thiobarbiturique (TBA) /malondialdéhyde (MDA).

| | ηmol MDA/ mg acide linoléique | % d'inhibition |
|---|---|---|
| Témoin(sans inhibiteur) | 7,95 | O |
| β-alanyl-histamine(25mM) | 4,25 | 47 |
| Carnosine(25mM) | 3,24 | 59 |
| Imidazole(25mM) | 6,95 | 11 |
| β-alanine(25mM) | 7,91 | 0 |
| Histamine(25mM) | 10,15 | -32 |
| β-alanine(25mM) + histamine(25mM) | 9,06 | -15 |

L'activité du type peroxydase des produits pseudo-dipeptides de l'invention s'exerce par la réduction des peroxydes de type L-OOH résultant de l'attaque des lipides membranaires par des radicaux oxygénés. Ces formes péroxydées peuvent se fragmenter, entraînant la rupture de la membrane cellulaire. Ce mode d'action est complémentaire de l'activité antiradicalaire permettant ainsi une désactivation particulièrement efficace du processus de dégradation des membranes biologiques. Ce mode d'action est appelé "activité peroxydase" par analogie avec certains

enzymes (catalases, glutathione-péroxydase) qui agissent sur le péroxyde d'hydrogène.

Un produit pseudo-dipeptide selon l'invention tel que la β-alanyl-histamine inhibe la réaction

$$\text{L-OOH} \rightarrow \text{produits de dégradation (tétradiènes, cétodiènes),}$$

en réduisant le péroxyde

$$\text{L-OOH} \rightarrow \text{L-OH}$$

Des tests effectués avec des composés peroxydés tels que le 13-monopéroxyde d'acide linoléique ou les hydropéroxydes de phosphatidylcholine montrent qu'un produit pseudo-dipeptide selon l'invention, la β-alanyl-histamine se comporte de façon comparable à la β-alanyl-histidine ou carnosine. Le comportement réducteur biologique de ce produit a été comparé à un agent réducteur chimique, le borohydrure de sodium qui inhibe la réaction de dégradation de façon identique.

Les tests suivants illustrent cette activité

PROTOCOLE 1

L'évolution des lipides péroxydés est évaluée à l'aide de 3 méthodes:

1°) mesure spectrophotométrique à 233 nm,

$$\varepsilon = 2{,}8\ 10^4\ M^{-1}cm^{-1}$$

(O.S. Privett, C. Nickell, W.O. Lundberg, and P.D. Bayer, dans J. Am. Oil Chem. Soc, Vol 32 (1955), p.505-511.

2°) Dosage iodométrique: méthode de M. Hicks et J.M. Gebicki, Analytical Biochemistry vol 99 (1979) p 249-253.

3°) chromatographie sur plaque silicagel (hexane/éther/acide acétique 8/7/0,1).

| | Activité "péroxydase" ηmol de L-OOH réduites/heure |
|---|---|
| Témoin (a) | 36,7 ± 14,7 |
| β-alanyl-histamine(10mM)(b) | 138,1 ± 18,7 |
| β-alanyl-histamine(20mM)(b) | 187,5 ± 15,7 |
| $NaBH_4$ (10mM) (c) | 312,5 ± 14,5 |

(a) 13-monopéroxyde d'acide linoléique 0,5 mM sans agent réducteur (préparation selon la méthode de H.W. Gardner (1975) Lipids, vol 10, p.248-252).

(b) activité entièrement inhibée par l'addition de 0,5 mM de EDTA.

(c) agent réducteur chimique.

PROTOCOLE 2

L'activité type "péroxydase" a également été évaluée sur un modèle "liposome". Les hydropéroxydes de phosphatidylcholine (PC-OOH) sont préparés selon un protocole similaire à celui déjà décrit pour le monopéroxyde d'acide linoléique.

La réduction des formes péroxydes est suivie en dosant les péroxydes restants selon une méthode établie (Hicks M and Gebicki J.M (1979), Anal. biochem., vol 99 p 249-253.

| | Nb moles de PC-OOH réduites/Nb moles de PC total x heure |
|---|---|
| β-alanyl-histamine(lOmM) | $0{,}77 \times 10^{-3}$ |
| L-carnosine(10mM) | $3{,}16 \times 10^{-3}$ |
| β-alanyl-histamine(25mM) | $1{,}56 \times 10^{-3}$ |

(suite)

| | Nb moles de PC-OOH réduites/Nb moles de PC total x heure |
|---|---|
| L-carnosine(25mM) | $3,65 \times 10^{-3}$ |

On s'aperçoit, à la lecture des tests ci-dessus que, dans certains cas, la L-carnosine donne des résultats supérieurs à la β-alanyl-histamine. Cependant, la désactivation enzymatique au niveau cutané de la L-carnosine ne permet pas d'espérer d'aussi bons résultats in-vivo.

Les pseudo-dipeptides selon l'invention peuvent agir plus en aval encore du phénomène de "stress oxydatif", par leur effet "anti-lipofushine". On l'a vu, la péroxydation des acides gras, et en particulier des phospholipides, conduit à leur fragmentation. Ces sous-produits de fragmentation sont toxiques, ils provoquent la réticulation des protéines. Les complexes lipoprotéiques pigmentés qui sont formés sont appelés "lipofushines"

La péroxydation des acides gras par les radicaux libres ne détruit pas seulement ces acides mais inactive les protéines avec formation de polymères "cross-linked". ce processus qui se fait au détriment à la fois des enzymes et des organites cellulaires, est considéré pour jouer un rôle dans le processus de vieillissement. Ces polymères dérivés d'une liaison croisée entre des acides gras polyinsaturés et des protéines sont appelés lipofuscines.

Les pseudo-dipeptides de l'invention fournis pour renforcer les défenses naturelles de l'organisme permettent de limiter la détérioration des protéines par ce processus en réduisant significativement la formation des complexes lipoprotéiques.

Un autre mode d'action des produits pseudo-dipeptides selon l'invention repose sur leurs propriétés anti-glycation, c'est-à-dire leurs capacités à inhiber la condensation spontanée entre les résidus aminés des protéines et les sucres réducteurs, et principalement le glucose.

La suite de réactions chimiques qui s'ensuit, était connue depuis des dizaines d'années par les chimistes de l'alimentation sous le nom de réaction de Maillard. Elle entraîne une accumulation de liaisons irréversibles entre les protéines, dont le nombre augmente avec l'âge. Il se forme des "liaisons croisées" entre des protéines telles que le collagène ou l'élastine (Ref.K. Reiser, R.J. McCormick, and R.B. Rucker - "Enzymatic and nonenzymatic cross-linking of collagen and elastin" - FASEB Journal, vol.6 (1992), p.2439-3449), avec pour conséquence, un raidissement et une rigidification des tissus, phénomènes caractéristiques des tissus vieillissants. Ce phénomène concerne surtout les protéines à longue durée de vie (protéines du cristallin, collagène, myéline), il est également responsable de la "cataracte du diabétique", individu chez qui la formation de protéines réticulées est fortement accélérée.

L'action antiglycation est due au fait que les produits pseudo-dipeptides selon l'invention sont des composés nucléophiles aminés qui s'opposent à la réticulation des protéines par les sucres, en intervenant au niveau des intermédiaires de la réaction de Maillard (voir plus haut). Dans les produits de l'invention c'est une amine nucléophile stériquement peu encombrée qui confère cette propriété.

Enfin, l'activité de régénération cellulaire dans le processus de cicatrisation a été démontré pour les produits pseudo-dipeptides selon l'invention. En effet, le processus de cicatrisation comporte trois phases: une phase vasculaire et inflammatoire, une phase proliférative ou il se produit une prolifération des fibroblastes et une néosynthèse du collagène, et une phase de remodelage au cours de laquelle il se produit un équilibre entre collagènolyse et biosynthèse. Dans ce processus, les produits pseudo-dipeptides agissent en tant qu'agents chimiotactiques ou activateurs qui provoquent l'arrivée vers le foyer cicatriciel des cellules participant à la reconstruction des tissus.

Un test de mise en évidence de l'activité d'un produit pseudo-dipeptide selon l'invention, la β-alanyl-histamine, sur la réparation du tissu conjonctif cutané a été pratiqué chez le rat. Cette expérimentation a été effectuée sur un lot de 12 rats Wistar. Une incision d'une longueur de 12 mm impliquant en profondeur l'ensemble épiderme et derme. A la fin de la cicatrisation les rats ont ensuite été traités quotidiennement par application topique de la β-alanyl-histamine pendant trois semaines. En même temps, un lot témoin de 12 rats a été traité avec un placébo pendant la même durée d'expérimentation.

Après les 3 semaines d'expérimentation, un prélèvement du tissu cicatriciel a montré que la cicatrice est peu ou pas visible en surface. L'épiderme est bien reconstitué. les couches cornées sont identiques à celles de l'épiderme normal voisin. Elles montrent un aspect variable suivant que l'on considère les animaux témoins ou les animaux traités.

La zone dermique en voie de cicatrisation apparaît comme une bande relativement étroite s'étendant de l'épiderme à la couche musculaire. La limite avec la zone non lésée est nette, sans aspect de transition. La densité et l'aspect des éléments du tissu conjonctif sont sensiblement les mêmes dans la couche capillaire et dans la couche profonde. Cependant, les fines fibres élastiques ou de réticuline sont généralement plus nombreuses dans les couches dermiques superficielles. La substance fondamentale est plus abondante que normalement comme le montrent les colorations par le bleu de toluidine (métachromasie à pH acide et technique de Lissberg).

Par contre les rats du lot témoin présentent un épiderme avec bourrelet cicatriciel d'une épaisseur supérieure à la normale. On note une hyperplasie fibroblastique et néovasculaire sur l'ensemble de la région cicatricielle. Les

fibroblastes sont en outre modérément hypertrophiés. Quelques rares faisceaux collagènes épais peuvent s'observer, mais la plus grande partie des faisceaux sont courts et d'épaisseur moyenne.

Selon un autre mode d'action, les produits pseudo-dipeptides selon l'invention favoriseraient la cicatrisation en agissant sur les cellules du système immunitaire impliquées à un stade précoce dans la cicatrisation (lymphocytes, mastocytes, monocytes) et dont le rôle principal est la sécrétaion des facteurs de croissance.

Ce pouvoir "immunostimulateur" est mis en évidence à l'aide d'un test in vitro sur splénocytes murins. Le protocole est extrait de : J. Kunert-Radek, H. Stepien, K. Lyson et M. Pawlikowski - "Effects of calcium channel modulators on the proliferation of mouse spleen lymphocytes in vitro" - Agents and Actions, vol.29, Nos 3-4 (1990), p. 254-258.

La prolifération cellulaire est suivie par la mesure du taux d'incorporation de thymidine tritiée dans les cellules, exprimé en nombre de désintégrations par mn, déduites du bruit de fond.

Les résultats obtenus avec un immunostimulant de référence, la concanavaline A, sont donnés à titre de comparaison.

L'effet immunostimulateur est exprimé par un Index de Stimulation (IS).

$$IS = \frac{\text{Nb de désintégrations par mn d'une suspension cellulaire additionnée de composé mitogène}}{\text{Nb de désintégrations par mn d'une suspension cellulaire de référence sans mitogène}}$$

Les valeurs correspondent à la moyenne de trois mesures.

On observe un effet immunostimulateur maximal pour une concentration de 25µg/ml en β-alanylhistamine. En conclusion, on observe un effet immunostimulateur (prolifération cellulaire) modéré pour ce pseudo-dipeptide pour des concentrations comprises entre 5 et 25 µg/ml.

| | Concentration Immunomodulateur (µg/ml) | | | | |
|---|---|---|---|---|---|
| | 2,5 | 5 | 10 | 25 | 50 |
| IS(β-alanyl-histamine) | 4,8 | 15,7 | 16,9 | 18,6 | 12,8 |
| IS (concanavaline A) | 4,8 | 25.1 | 20.5 | 12,6 | |

Cette activité est comparable à celle d'un mitogène de référence, la concanavaline A.

Les produits pseudo-dipeptides selon l'invention peuvent participer à la protection de l'organisme contre les réactions allergiques, et notamment s'opposer au choc anaphylactique, en exercant une activité régulatrice de la réponse immunitaire.

Ce type de produit agirait également en s'opposant à l'augmentation de la perméabilité des vaisseaux sanguins, responsables de l'oedème et par un effet antagoniste vis-à-vis de la bradykinine, un effecteur de la réaction oedèmateuse (Ref. K. Nagai Ed. - "Studies of carnosine and related chemicals on the physiology of wound repair mechanism", pages 24-26, Tokyo (1976)). c'est le caractère anti-histaminique potentiel de cette famille qui jouerait un rôle dans cette propriété.

## Applications thérapeutiques et cosmétologiques

L'ensemble des propriétés énoncées ci-dessus, dont la caractéristique essentielle est de participer au renforcement des moyens naturels de défense et de réparation de l'organisme conduit à des applications thérapeutiques et cosmétologiques appréciables des produits pseudo-dipeptides selon l'invention.

Les propriétés antioxydantes des pseudo-dipeptides selon l'invention permettent de proposer ces produits pour le traitement des pathologies associées au "stress oxydatif".

Une application thérapeutique importante est le traitement de la cataracte. Les causes des différentes cataractes sont diverses. Les mécanismes impliqués dans ces pathologies, qu'elles soient du type "cataracte sénile" ou "cataracte du diabétique" sont regroupés en deux catégories: les mécanismes oxydatifs (M. A. Babizhayev, A.I. Deyev, L.F. Lindberg, "Lipid peroxidation as a possible cause of cataract", Mechanisms of Ageing Dev., Vol 44 (1988); P. 69-89), et les mécanismes de réticulation du type glycation (T.J. Lyons, G. Silvestri, J.A. Dunn, D.G.Dyer, J.W. Baynes "Role of glycation of lens crystallins in diabetic and non-diabetic senile cataracts", Diabetes Vol. 40, N° 8, (1991), P. 1010-1015).

Comme on l'a vu précédemment, les propriétés antioxydantes des pseudo-dipeptides s'exerçant en particulier par leur activité anti-radicaux libres et type "péroxydase" et également leur activité anti-glycation, font des produits

pseudo-dipeptides selon l'invention des produits efficaces pour soigner la cataracte.

Les produits pseudo-dipeptides selon l'invention peuvent s'opposer aux phénomènes oxydatifs responsables de l'athérosclérose. Dans cette pathologie, l'oxydation de particules lipoprotéiques de faible densité (LDL) circulant dans le sang est responsable de la fragmentation de la partie protéique (apoprotéine B) comme de la fraction lipidique de ces particules. Les fragments formés induiraient l'apparition de formes cellulaires anormales (monocytes et macrophages chargés en cholestérol) capables de s'agréger sur les parois des vaisseaux sanguins et de former la plaque d'athérome.

Les produits pseudo-dipeptides selon l'invention seraient de plus, particulièrement adaptés au traitement de cette maladie dans la mesure où il a été mis récemment en évidence que des phénomènes de glycation sont également impliqués dans la genèse de la plaque d'athérome (Ref. T.J. Lyons - "Glycation and oxidation - A role in the pathogenesis of Atherosclerosis" - American Journal of Cardiology, vol.71, N' 6 (1993), p. 1326-1331).

Les produits pseudo-dipeptides selon l'invention peuvent également s'opposer au processus de cancérogénèse dans la mesure où il a été démontré que les espèces radicalaires dérivées de l'oxygène sont responsables de la coupure ou de la modification des brins d'ADN, ces transformations pouvant être à l'origine de l'évolution des cellules saines en cellules cancéreuses.

De même, les propriétés antioxydantes des produits pseudo-dipeptides selon l'invention permettent d'indiquer ces produits pour le traitement des états inflammatoires pathologiques, et en particulier pour le traitement de l'arthrite rhumatisante. En effet, la détérioration du liquide synovial est un symptôme caractéristique de l'arthrite de type inflammatoire et on a pu montrer que la dégradation d'un de ses constituants essentiels, l'acide hyaluronique, était dû à un "stress oxydatif". Des études plus récentes (Ref. B. Halliwell and J.M.C. Gutteridge-"Chronic inflammation and the auto immune deseases" - Free Radicals in Biology and Medecine - B. Halliwell and J.M.C. Gutteridge Eds - Clarenton Press (1989), Oxford, p. 422-438) ont également mis en cause des phénomènes de péroxydation lipidique mis en jeu dans ce processus et qui expliqueraient l'action bénéfique des produits selon l'invention.

Les propriétés antioxydantes des produits pseudo-dipeptides selon l'invention peuvent également être exploitées en traitement annexe d'une radiothérapie. Cet effet radioprotecteur, déjà connu pour la β-alanyl-histidine repose sur les propriétés cytostimulantes de ce type de composé, notamment vis-à-vis des cellules de la moelle osseuse, très sensibles aux rayonnements utilisés en radiothérapie.

Selon des données récentes, certains symptômes épileptiques pourraient provenir de lésions provoquées par les radicaux libres oxygénés sur certaines régions du cerveau (Ref. G.R. Jackson, K. Werrbach-Perer, J.R. Perez-Polo-"Role of nerve growth factor in oxidant-antioxidant balance and neuronal injury - II - A conditioning lesion paradigon"-Journal of Neuroscience Research, vol.25, N°3 (1990), p.369-374). Le pouvoir régénérant des tissus (nerveux en l'occurence) des produits de l'invention est un élément important dans cette pathologie associée à une dégénérescence du tissu nerveux. Ces produits pourraient également être indiqués pour le traitement de la maladie de Parkinson dans laquelle serait impliqué un "stress oxydatif" touchant le tissu cérébral.

Au niveau de la peau, les propriétés antioxydantes des produits pseudo-dipeptides de l'invention peuvent être exploitées pour neutraliser les effets des espèces radicalaires oxygénées générées par le rayonnement lumineux. Ils s'opposeront ainsi efficacement aux réactions photo-allergiques (les espèces radicalaires induisent au niveau de la peau la formation de molécules allergisantes). En association avec un principe actif sensible à l'oxydation (ex: Chlorpromazine), ils préviendront sa transformation en un composé toxique.

Ce principe trouve sa meilleure application lors des traitements par photochimiothérapie de certaines maladies de la peau. En effet ces traitements reposent sur l'utilisation d'un photosensibilisant (ex: psoralène) qui, sous l'effet d'un rayonnement, exerce une action bénéfique (interaction avec l'ADN), mais qui, malheureusement, s'accompagne de la formation d'espèces radicalaires, responsables d'effets secondaires indésirables.

Les produits de l'invention peuvent également être indiqués pour s'opposer à l'apparition de symptômes cutanés chez des individus souffrant de porphyrie, car les porphyrines potentialisent les dommages occasionnés par les R.L.

Ils pourront également s'opposer à la formation de lésions cutanées liée au "stress oxydatif" chez les personnes souffrant de maladies autoimmunes tel que le Lupus Erythémateux Chronique (SLE).

Ils s'opposeront également efficacement aux effets du "coup de soleil": érythèmes, oedèmes et formation de cellules caractéristiques au niveau de la peau.

Les propriétés antioxydantes des composés selon l'invention peuvent bien sûr être utilisées pour la prévention du vieillissement cutané. Les arguments théoriques et expérimentaux qui justifient cette approche ont déjà été donnés précédemment.

Enfin, il a été démontré expérimentalement que ces composés s'opposaient à d'autres phénomènes caractéristiques du tissu cutané vieillissant :

- La réticulation non-enzymatique de protéines telles que le collagène ou l'élastine par les sucres (V.M. Monnier-"Nonenzymatic glycosylation, the Maillard reaction and the aging process" - Journal of Gerontology, vol. 45, N°4

(1990), p. B105-111),
- La formation de complexes lipoprotéiques (lipofushines).

Une autre série d'applications relève des propriétés cytostimulantes des produits pseudo-dipeptides selon l'invention et, comme il l'a été démontré, de leurs propriétés immunostimulatrices. Ces propriétés permettent de favoriser la régénération tissulaire et la cicatrisation, et d'une manière générale, de réguler les fonctions faisant intervenir les effecteurs de la réponse immunitaire.
Ils peuvent ainsi être utilisés pour favoriser la régénération des tissus conjonctifs cutanés perturbés. Ils favorisent la réparation des muqueuses après brûlures ou après traitements chimio et radiothérapiques.
Selon ce principe, ces produits peuvent également être indiqués pour la prévention et le traitement des rides.
Les propriétés cytostimulatrices et régénératrices des peptoïdes s'exercent tout particulièrement vis à vis des tissus musculaires où l'on rencrontre des concentrations élevées d'un dipeptide naturel apparenté, la β-alanyl-histidine. Bien que le rôle physiologique de ce composé n'est pas, à l'heure actuelle, parfaitement établi, il est étroitement lié au métabolisme musculaire. Ainsi, les produits de l'invention pourraient participer à l'amélioration de la contractilité des muscles, réguler la contraction cardiaque. On peut également utiliser ce type de propriétés pour le traitement de certaines dégénérescences musculaires telles que la myodystrophie de Dushen. Les propriétés cicatrisantes des produits pseudo-dipeptides selon l'invention trouvent leur application dans de nombreux domaines. On peut citer le traitement des ulcères gastriques, pour lequel un produit dipeptide apparenté à la carnosine, a donné de bons résultats (M. Ito, T. Tanaka and Y. Suzuki-"Effect of N-(3-aminoproprionyl)-L-histidinato zinc (Z-103) on healing and hydrocortisone-induced release of acetic acid ulcers in rats with limited food-intake-time" - Japaneese Journal of Pharmacology, vol.54 (1990), p.513-521). En outre, les propriétés antioxydantes et anti-inflammatoires des produits de l'invention sont utiles pour le traitement de cette pathologie.
Les propriétés cicatrisantes des produits pseudo-dipeptides selon l'invention sont également particulièrement indiqués pour le traitement des atteintes cornéennes. Ils pourront être administrés en traitement post-opératoire, après incision de la cornée pour la correction de la myopie, par exemple.
Ils peuvent également être avantageusement utilisés pour le traitement des pathologies de "l'oeil sec" en favorisant la cicatrisation de l'épithélium cornéen mais également grâce à un comportement de "larme artificielle": protection des tissus lésés contre le "stress oxydatif" (état, inflammation, rayonnement U.V.), incorporation dans des formulations favorisant la restauration du film lacrymal.
Des compositions contenant les produits pseudo-dipeptides de l'invention pourront aussi, de par leur action immunostimulante et régénératrice des tissus, s'opposer aux phénomènes de dégénérescence rétinienne. Ils verront leurs effets renforcés par le fait qu'une composante "stress oxydatif" intervient dans cette pathologie (Ref. R.E. Anderson, L.M. Rapp, R.D. Wiegand - Current Eye Research, vol.3 (1984), p.223-227).
Une autre application importante des produits pseudo-dipeptides de l'invention est plus spécifiquement en relation avec leurs propriétés immunomodulatrices. Il pourrait ainsi être particulièrement bénéfique d'inclure les produits pseudo-dipeptides selon l'invention dans les parfums et déodorants afin de s'opposer aux réactions allergiques, et en particulier aux chocs anaphylactiques, provoqués par certaines compositions fortement odorantes.

## Applications comme stabilisant et conservateur

L'excellente tolérance vis-à-vis des pseudo-dipeptides selon l'invention, et leurs propriétés antioxydantes et cytostimulantes modérées permettent de proposer ces produits pour la conservation et la protection de substances sensibles à l'oxydation, de matières alimentaires ou d'organes et de tissus conservés ex vivo.
On peut citer la prévention de l'oxydation des liposomes afin d'améliorer leur stabilité et d'éviter la formation de sous-produits toxiques, la protection de l'acide hyaluronique incorporé dans des formules cosmétiques contre l'action dépolymérisante des radicaux libres, la protection des huiles et des produits alimentaires oxydables , de produits diététiques.
Les produits de l'invention permettent d'améliorer la conservation des vaccins du sang et du serum, la préservation d'organes voués à la transplantation (avec une référence particulière pour le coeur).
Les applications des produits pseudo-dipeptides de l'invention ont été mises en évidence dans les exemples suivants qui donnent les doses et les formes administrables des produits utilisés comme médicaments ou comme agents stabilisants.

### Exemple 1

Cet exemple concerne l'utilisation témoin de la la β-alanyl-histamine pour soigner des patients atteints de cataracte.
L'étude a été conçue comme une évaluation prospective des modifications de l'opacité du cristallin chez les

patients atteints de la cataracte. Tous les patients ont subi un examen ophtalmique initial. celui-ci incluait l'établissement de l'historique médical du patient, une mesure de l'acuité visuelle à correction maximale, des ophtalmoscopies directes et indirectes, une évaluation des opacités avec la lampe à fente, une classification clinique du cristallin à mydriase maximale, une classification selon le type de cataracte, et des photographies du cristallin par rétroillumination. De plus, des échantillons de sang ont été prélevés au départ, puis en suivi pour les analyses chimiques. L'ensemble a permis d'établir l'état initial des patients avant traitement (définition d'une "ligne de base").

Des suivis d'examens ophtalmiques furent effectués toutes les 2 semaines pendant 2 mois, puis chaque mois. Ils incluaient l'acuité visuelle maximale après correction, des ophtalmoscopies directes et indirectes, un examen à la lampe à fente, et après une dilatation maximale de la pupille (en excluant les glaucomes primaires à angle ouvert), une classification clinique des cristallins, une classification de la cataracte. Ont été prises également des photographies du cristallin observé par rétroillumination ou avec la lampe à fente.

Parallèlement, les changements du cristallin ont été examinés chez tous les patients en utilisant le microscope à la lampe à fente de Zeiss. Les observations photographiques obtenues avec cette technique incluaient un examen initial, des études intermédiaires et un examen final à la fin de la période de traitement.

En utilisant la technique de la lampe à fente, le cristallin a été photographié couche par couche afin de visualiser tout le segment antérieur de l'oeil, de la cornée à la capsule postérieure

Avec cette technique, un examen réflexe à la lumière rouge a été réalisé, selon une technique de retroillumination conventionnelle. Les modifications du cristallin ont par la suite été classées selon la localisation anatomique et l'importance de la modification.

Des opacités corticales ont été évaluées en rétroillumination et photographiées. Le film a ensuite été soumis à une mesure de densitométrie linéaire avec un microdensitomètre. Les valeurs d'opacité ont été reportées en valeur relative en utilisant une gamme d'opacification étalon. Les mesures d'opacité corticale ont été étalonnées en tenant également compte de la surface totale exposée au rayonnement. Les opacités sub-oculaires postérieures ont été mesurées en rétroillumination par la plus grande hauteur, de la base au sommet, et la plus grande largeur, en utilisant l'échelle de la lampe à fente.

Des photographies du cristallin furent aussi prises sur des opacités nucléaires et corticales et par la suite furent graduées.

Les différents clichés pris avec la lampe à fente ont été analysés à l'aide d'un "tube plumbicon". Cet appareil permet de transposer avec une grande fidélité une mesure d'intensité lumineuse en un signal électrique qui est entré et stocké dans un système d'analyse d'image informative. A l'aide d'un programme spécifique, l'image du cristallin ainsi "numérisée" peut être reproduite sous la forme de graphes à 2 ou 3 dimensions.

Une description détaillée de cette méthode de traitement de l'image est donnée dans l'article de Babizhayev M.A. Zhukotskii A.V. and Sologub A.A. (1992), "Image analysis of the lens opacities induced in developing chick embryo by glucocorticoïd", Exp. Eye Res. 55, 521-537.

Des exemples de ces images reconstruites sont fournies en Annexe 1. Les histogrammes représentés montrent sur un exemple concret l'intérêt d'une telle méthode d'évaluation. Un cas de cataracte subcapsulaire postérieure est étudié avant et après 2 mois et demi de traitement en administration topique (instillations) avec un collyre à 1% de $\beta$-alanyl-histamine. Les histogrammes donnent la distribution quantitative des opacités avec un pic d'intégration de $58,44 \pm 18,14$ avant traitement, et $44,95 \pm 8,77$ après 2,5 mois de traitement.

Ces analyses révèlent une régression partielle des opacités dans la région postérico-subcapsulaire du cristallin.

Les résultats obtenus avec cette méthode sont en accord avec les observations cliniques classiques.

Dans cette étude, 11 a été déterminé différents groupes, à savoir:

un groupe 1 de référence, avec des patients non traités (5 patients, 7 yeux),

un groupe 2, auquel il a été administré un collyre qui était un soluté isotonique contenant 1% de $\beta$-alanyl-histamine à pH 7,4. Il a été procédé à deux administrations locales par jour (10 patients, 15 yeux)

un groupe 3, qui a été soumis au même traitement que le groupe 2, stimulé par un traitement d'attaque durant les deux premières semaines, à savoir: 2 injections sous conjonctivales 2 fois par semaine, de 0,10 et de 0,15 ml du soluté défini pour le groupe 2 (3 patients, 4 yeux)

un groupe 4, qui a reçu une administration locale sous forme de collyre d'un soluté isotonique deux fois par jour (5 patients, 7 yeux), le groupe 4 étant comme le groupe 1, un groupe de référence.

Suivant le protocole décrit plus haut, on détermine des valeurs de la densité optique du cristallin en fonction de son opacité, ainsi que l'acuité visuelle, et le tout sur 24 mois. En prenant ces deux paramètres, on obtient les tableaux donnant les résultats concernant la modification de l'acuité visuelle au bout de 24 mois en annexe 2, et les résultats concernant la modification de l'opacité visuelle au bout de 24 mois en annexe 3.

On peut conclure que suivant les deux paramètres observés, l'administration du produit est très significative

dans l'amélioration de la vision et la régression de la cataracte.

De la même façon, on a expérimenté une forme de collyre à base de N-acétyl-β-alanyl-histamine à raison de 1% dans un soluté isotonique, qui donne des résultats conformes au but de l'invention. Avec une seule administration locale quotidienne, les résultats ont été superposables à ceux obtenus précédemment. Ce constat peut être expliqué par une meilleure biodisponibilité de la forme acétylée.

Bien que l'exemple ci-dessus fait état de collyre, on peut administrer le produit selon l'invention sous la forme de toute formulation oculaire communément acceptée telle qu'un gel ou autre (voir réf. "Remington's parmaceutical sciences handbook" Hack Pub. Co. USA contenat de 1 à 100 mM de β-alanyl-histamine.

**Exemple 2**

On a mis en évidence l'activité réparatrice sur les muqueuses des produits selon l'invention en utilisant des formulations à de très faibles doses. On a pu étudier cette action sur des gingivo-stomatites d'origines diverses (port de prothèses dentaires mal adaptées, administration par erreur de liquide corrosif, et cobaltothérapie de la sphère-oropharingée).

Dans tous les cas, les causes de la formation et détérioration des muqueuses gingivales et buccales ont été stoppées. En plus des traitements classiques adaptés et administrés systématiquement, il a été remis à 50% des patients, un gel aqueux aromatisé à l'orange contenant 0,28% de N-acetyl-3-phenyl-3-aminopropionyl histamine. Les observations sur ces différents cas ont montré une excellente tolérance et une réduction significative du temps réparateur allant de 20 à 55%.

On peut conclure à une activité positive en respectant la dose de formulation.

**Exemple 3**

On a pu mettre en évidence les propriétés cicatrisantes en utilisant une crème cicatrisante de composition:

Nanosphères (diamètre 100nm) contenant 5% de γ-amino butyryl-histamine.....10g
Excipient hydrodispersible qsp 100g pH 6,5

Cette crème a été appliquée deux fois par jour par légers effleurages sur des cicatrices fermées. Les zones cicatricielles et péri-cicatricielles ont été massées jusqu'à pénétration de la pommade.

Les cas étudiés étaient trois cicatrices de moins de 14 mois post-chirurgicales, dont deux avec chéloides et cinq séquelles acnéiques

Dans tous les cas, il a été observé une amélioration significative de la cicatrice, une réduction des bourrelets et infractuosités de la peau jusqu'à disparition totale dans deux cas.

En parallèle, il a été noté une restauration d'un teint uniforme avec disparition des turgescences. Ces résultats sont significativement positifs.

**Exemple 4 (Témoin)**

Le collyre suivant a été utilisé pour le traitement de lésions de la cornée et en particulier sur une pathologie de "l'oeil sec".

| | |
|---|---|
| Liposomes de phosphatidylcholine | 8 mg/ml |
| Fibronectine de plasma humain | 10 μg/ml |
| β-alanyl-histamine | 25 μg/ml |

dans un milieu tamponné à pH compris entre 6,2 et 7,4

La fibronectine et la β-alanyl-histamine sont encapsulés dans les liposomes.

La fibronectine est une molécule d'attachement cellulaire qui va favoriser la cicatrisation et la restauration du film lacrymal.

Le collyre est appliqué à la surface de l'oeil pendant 10 jours à raison de deux instillations par jour. La quantité appliquée doit être suffisante pour former un film continu à la surface de l'oeil.

Résultats:

La formule reconstitue durablement une couche protectrice à la surface de l'oeil, comme le met en évidence

une étude de rétention sur la cornée d'un marqueur fluorescent (protocole extrait de: R.F. Barber, P.N. Shek "Liposomes and tear film .1. Release of vesicle entrapped carboxyfluorescein" - Biochimica Biophysica Acta: lipids and lipid metabolism, vol. 879 (L81), n°2 (1986), p. 157-163).

La cicatrisation est suivie à l'aide d'une lampe à fente: on observe une rapide reconstitution de l'épithélium cornéen.

**Exemple 5**

La pommade utilisée dans cet exemple a également été utilisée pour le traitement des brûlures de 1° et 2° degré superficiel.

| | |
|---|---|
| acide parahydroxycinnamique | 0,1 g |
| essence de menthe | 0,3 g |
| essence de lavande | 0,5 g |
| menthol nanosphérisé | 0,05g |
| B-aminoisobutyryl-histamine | 0,5 g |
| excipient hydrodispersible qsp | 100 g |

Dès l'apparition de la brûlure, on applique la pommade en fine couche. On répéte l'application toutes les heures dans un premier temps, jusqu'à cessation de la douleur. Sur les brûlures du 1er degré, l'érythème disparaît rapidement. Sur les brûlures du 2ème degré superficielles avec flyctènes et oedème sur épiderme rougi suivant la surface brûlée, on observe une résorption de l'oedème dans les 2 heures, une régression de la douleur dans les 3 ou 4 heures.

Dans les deux à trois jours qui suivent, les tissus sous-jacents sont régénérés plus rapidement. Il est possible de libérer les vésicules du liquide retenu par ces dernières avec formation d'une nouvelle couche épidermique et élimination des tissus morts. On note une accélération de la réparation épidermique, d'une part, et une excellente qualité, d'autre part.

**Exemple 6**

L'activité antiradicalaire des produits pseudo-dipeptides de l'invention a été mise en évidence dans l'exemple suivant de formulation cosmétique utilisable comme lotion after-shave:

| | |
|---|---|
| 6-aminocaproyl-histamine nanosphérisé à 5% | 16 g |
| excipient liquide parfumé hydroalcoolique 6 qsp | 100 g |

une application régulière après rasage permet de conserver une peau en bon état.

**Exemple 7**

En se basant sur la même activité que l'exemple ci-dessus, une crème solaire a été réalisée avec la formulation suivante:

| | |
|---|---|
| 3 phényl-3-aminopropionyl-histamine | 1g |
| excipient hydrolipidique microdispersé qsp | 100g |

Différents cas ont été étudiés dans les salons esthétiques lors de séances d'irradiation UVB. Pour des hâles équivalents, lorsque l'on applique la crème après exposition, l'épiderme présente une meilleure qualité dans les jours qui suivent les irradiations.

**Exemple 8**

Toujours basée sur la même activité, on a réalisé une crème entretien visage de composition suivante:

| | |
|---|---|
| solution de 5-aminovaleryl histamine nanosphérisée à 4%<br>ion Cu++,Fe++ (sous forme d'acétylméthionine) | 20g |

(suite)

| | |
|---|---|
| excipient hydrodispersible qsp pH 6,5 | 100g |

Comme crème antivieillissement, avec deux applications quotidiennes, on a noté une régression des ridules, une peau plus souple et plus colorée.

Comme crème d'entretien réparatrice des agressions diverses, une application quotidienne suffit. On applique la crème par massage jusqu'à absorption totale, les Nanosphères vont former un film homogène avec libération prolongée. Un maquillage est tout à fait possible après l'application de cette crème.

On observe au niveau de la peau du visage, des résultats visibles stimatisés par les critères dits de bonne santé, souplesse, éclat, couleur uniforme et bonne hydratation.

**Exemple 9**

Les produits pseudo-dipeptides peuvent aussi être utilisés pour protéger des huiles cosmétiques facilement oxydables, riches en acides gras insaturés formulées en cosmétique ou diététique.

Il a été nécessaire d'ajouter 15 à 25 mmol par litre d'huile de produits pseudo-dipeptides de l'invention selon la formulation suivante:

20 mmol de β-alanyl-histamine acétylée

huile extraite de micro algues cultivées en eau de mer très riches en acides gras insaturés, dont EPA qsp pour 1 1 On a comparé le vieillissement de cette huile protégée à l'huile non protégée au bout d'un mois par le dosage de la malondialdéhyde. Pour l'huile non protégée, on observe une augmentation importante de la teneur en dialdéhyde, ce qui prouve bien l'activité de la β-alanine histamine acétylée dans la protection contre l'oxydation.

**Exemple 10**

Selon le même principe que précédemment on a préparé l'huile cosmétique suivante

25 mmol de 8-amino-octanoyl histamine huile de rosa mosqueta qsp pour 1 1 On a comparé les produits huile protégée/huile non protégée au bout de 5 semaines par la mesure de l'indice d'acide. Le dosage de ce dernier, dans ce cas présente une augmentation significative (valeur supérieure à 10 pour l'huile non protégée). On peut donc en conclure que la présence de 8-amino-octanoyl histamine a une action antioxydante sur l'huile de rosa mosqueta.

Tous les exemples ci-dessus ont montré que les pseudo-dipeptides selon l'invention sont actifs dans une fourchette de 1 à 100 millimoles d'actifs par litre avec un pic principal de 10 à 25 mM.

ANNEXE 1

DISTRIBUTION QUANTITATIVE DE l'OPACITE

**AVANT**

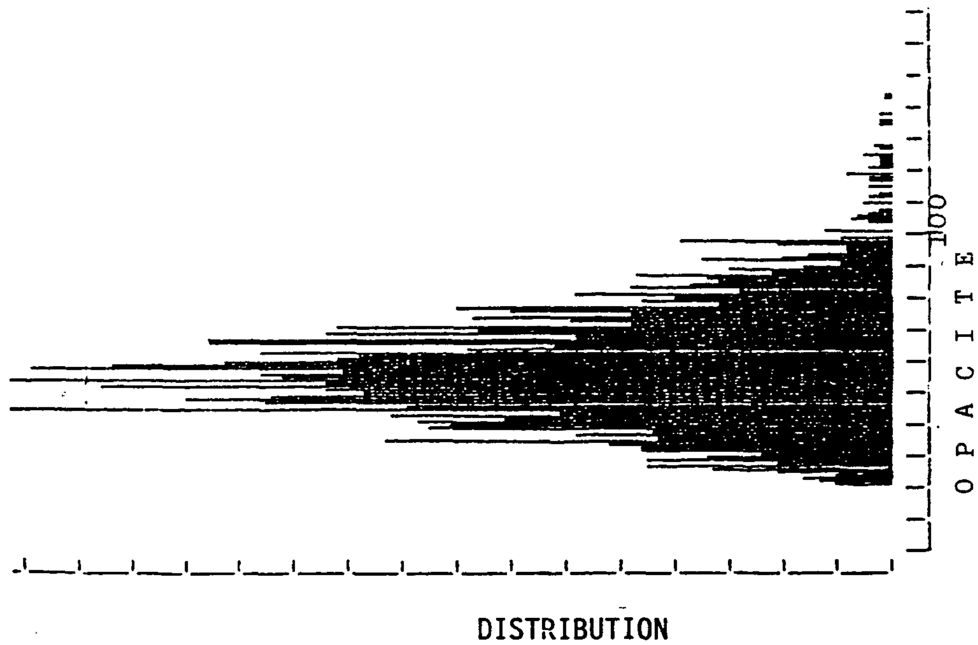


**APRES**

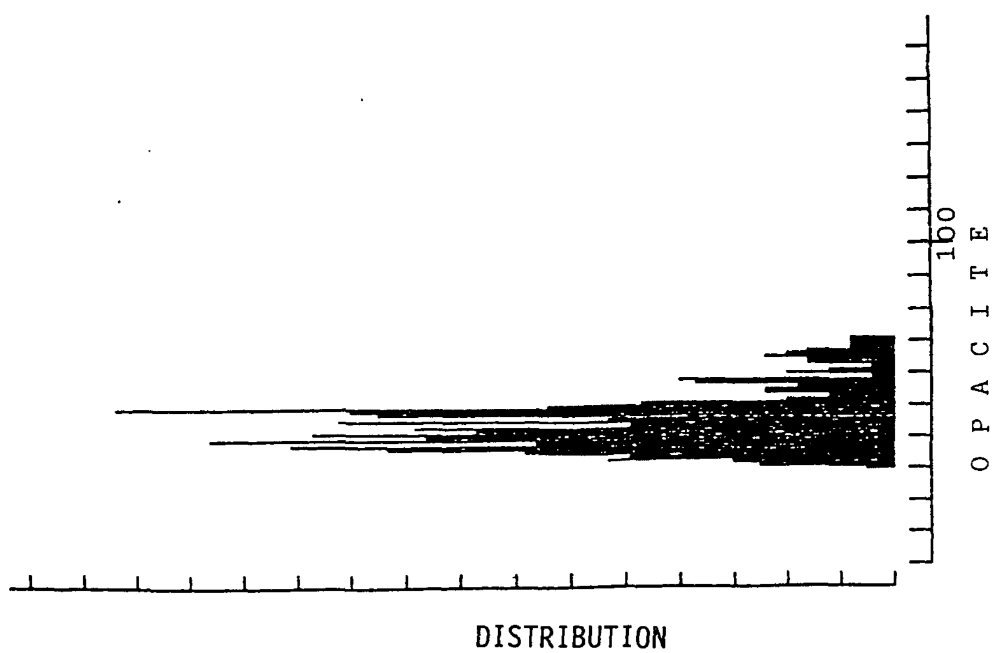

ANNEXE 2

MODIFICATION DE L'ACUITE VISUELLE AU BOUT DE 24 MOIS

TABLEAU 1

| PATIENTS NON TRAITES et TRAITES avec PLACEBO (Groupe 1 et 4) | | | | | | | | |
|---|---|---|---|---|---|---|---|---|
| Acuité visuelle de départ | Nombre de yeux | AMELIORATION de L'ACUITE VISUELLE | | | ETAT STATIONNAIRE | DETERIORATION DE L'ACUITE VISUELLE | | |
| | | + | ++ | +++ | | + | ++ | +++ |
| 20/25 | 2 | - | - | - | | - | 2 | - |
| 20/30 | 1 | 1 | - | 0 | | - | - | - |
| 20/40 | 3 | - | - | - | | 1 | 1 | 1 |
| 20/50 | 1 | - | - | 0 | | - | - | 1 |
| 20/60 | 2 | - | - | - | | 1 | - | 1 |
| 20/70 | 1 | - | - | 0 | | - | 1 | - |
| 20/100 | 0 | - | - | - | | - | - | - |
| 20/200 | 3 | 1 | - | - | | - | 1 | 1 |
| < 20/200 | 1 | - | - | - | | - | - | 1 |
| | 14 | 2 | 0 | 0 | | 2 | 5 | 5 |

TABLEAU 2

| PATIENTS TRAITES (Groupe 2 et 3) | | | | | | | | |
|---|---|---|---|---|---|---|---|---|
| Acuité visuelle de départ | Nombre de yeux | AMELIORATION de L'ACUITE VISUELLE | | | ETAT STATIONNAIRE | DETERIORATION DE L'ACUITE VISUELLE | | |
| | | + | ++ | +++ | | + | ++ | +++ |
| 20/25 | 1 | 1 | - | - | | - | - | - |
| 20/30 | 5 | 1 | 4 | - | | - | - | - |
| 20/40 | 4 | 1 | 3 | - | | - | - | - |
| 20/50 | 2 | 1 | 1 | - | | - | - | - |
| 20/60 | - | - | - | - | | - | - | - |
| 20/70 | 2 | 1 | 1 | - | | - | - | - |
| 20/100 | 4 | - | 1 | 2 | | - | 1 | - |
| 20/200 | 1 | - | 1 | - | | - | - | - |
| < 20/200 | 0 | - | - | - | | - | - | - |
| | 19 | 5 | 11 | 2 | | 0 | 1 | 0 |

ANNEXE 3

MODIFICATION DE L'OPACITE VISUELLE AU BOUT DE 24 MOIS

TABLEAU 1

| PATIENTS NON TRAITES et TRAITES avec PLACEBO (Groupe 1 et 4) | | | |
|---|---|---|---|
| % DE MODIFICATION | DIMINUTION DE L'OPACITE | ETAT STATIONNAIRE | AUGMENTATION DE L'OPACITE |
| 5 à 9% | - | - | 8 |
| 10 à 14% | - | - | 2 |
| 15 à 19% | 1 | - | 2 |
| 20 à 24% | - | - | 0 |
| + 25% | - | - | 1 |
| | 1 | - | 13 |

TABLEAU 2

| PATIENTS TRAITES (Groupe 2 et 3) | | | |
|---|---|---|---|
| % DE MODIFICATION | DIMINUTION DE L'OPACITE | ETAT STATIONNAIRE | AUGMENTATION DE L'OPACITE |
| 5 à 9% | 3 | - | - |
| 10 à 14% | 5 | - | - |
| 15 à 19% | 5 | - | - |
| 20 à 24% | 1 | - | - |
| + 25% | 5 | - | - |
| | 19 | - | - |

## Revendications

**1.** Produit de couplage entre l'histamine ou l'histamine méthyl-substituée et un acide aminé de formule :

$$X-NH-\overset{R_1}{\overset{|}{CH}}-\overset{R_2}{\overset{|}{CH}}-(CH_2)_p-COOH$$

dans laquelle :

- X représente un atome d'hydrogène ou un groupe acétyle ;
- $R_1$ représente un atome d'hydrogène ou un groupe phényle ;
- $R_2$ représente un atome d'hydrogène ou un groupe méthyle ;
- p est un nombre entier compris entre 0 et 3 inclus ;

  la liaison covalente avec l'histamine ou l'histamine méthyl-substituée étant une liaison peptidique entre le groupe carboxylique de l'acide aminé et le groupe amine de l'histamine ;
  ledit produit étant différent de la β-alanyl-histamine ou de la 6-aminocaproyl-histamine.

**2.** Produit selon la revendication 1, dans lequel ledit acide aminé est choisi parmi l'acide γ-aminobutyrique, l'acide β-aminoisobutyrique, l'acide 5-aminovalérique, l'acide 3-phényl-3-aminopropionique, la N-acetyl-β-alanine, l'acide N-acétyl-3-phényl-3-aminopropionique ou l'acide DL-β-aminobutyrique.

**3.** Produit selon la revendication 1 ou 2, qui est la N-acétyl-β-alanyl-histamine.

**4.** Procédé chimique de préparation du produit selon l'une des revendications 1 à 3, à partir dudit acide aminé et de l'histamine ou de l'histamine méthyl-substituée par les étapes suivantes :

- on rend ledit acide aminé N-protégé par un groupement X,
- on rend ledit acide aminé O-activé par estérification de la fonction carboxylique dudit acide aminé par réaction avec un composé choisi dans le groupe consistant en : alcool de cyanométhyle, o-nitrophénol, 2,4,5-trichlorophénol, p-nitrophénol, 2,4-dinitrophénol, pentachlorophénol, pentafluorophénol, N-hydroxyphtalimide, N-hydroxysuccinimide, 1-hydroxypipéridine et 5-chloro-8-hydroxy-quinoline.
- on couple ledit acide aminé N-protégé et O-activé avec l'histamine ou l'histamine méthyl-substituée, et
- on élimine X ou non selon le produit utilisé.

**5.** Procédé enzymatique de préparation du produit selon l'une des revendications 1 à 3, qui consiste à coupler l'acide aminé avec l'histamine ou l'histamine méthyl-substituée en présence d'un catalyseur enzymatique du type hydrolase.

**6.** Procédé selon la revendication 5 dans lequel ledit catalyseur enzymatique est une hydrolase choisie dans le groupe consistant en lipases extraites de micro-organismes ou d'origine animale, et en protéases.

**7.** Procédé selon la revendication 5 ou 6 comprenant les étapes suivantes :

- on rend l'acide aminé N-protégé par un groupement X,
- on rend l'acide aminé O-activé par un groupe Y,
- on couple l'acide aminé N-protégé et O-activité avec l'histamine ou l'histamine méthyl-substituée en présence dudit catalyseur, et
- on élimine le groupe X selon le produit utilisé.

**8.** Procédé selon la revendication 7 dans lequel l'acide aminé est rendu O-activé par estérification de la fonction carboxylique dudit acide aminé.

**9.** Procédé selon la revendication 8 dans lequel l'étape d'estérification pour rendre l'acide aminé O-activé est réalisée par la réaction avec un composé choisi dans le groupe consistant en alcools aliphatiques ou alcools aromatiques en excluant les alcools tertiaires, et comprenant notamment l'éthanol, les halogéno-alkylalcools tels que 2,2,2-trichloro-éthanol, le phénol, l'alcool de cyanométhyle, l'o-nitrophénol, le 2,4,5-trichlorophénol, le p-nitrophénol, le 2,4-dinitrophénol, le pentachlorophénol, le pentafluorophénol, le N-hydroxyphtalimide, le N-hydroxysuccinimide, la 1-hydroxypipéridine et la 5-chloro-8-hydroxy-quinoline.

**10.** Procédé selon l'une quelconque des revendications 5 à 9 dans lequel ledit produit est la N-acétyl-β-alanyl-histamine obtenue par les étapes suivantes :

- 2,38 g (15 mmoles) de N-acétyl-β-alanine éthyl ester et 1,11 g (10 mmoles) d'histamine sont dissous dans 40 ml d'un mélange tert-amylalcool-heptane (25:75),

- 0,5 g de lipase Amano- P (Pseudomonas sp.) sont ajoutés et la suspension est chauffée à 40°C, sous agitation jusqu'à disparition complète de l'histamine,
- la lipase est récupérée par filtration,
- le solvant de tert-amylalcool-heptane est évaporé sous vide pour donner un composé huileux, et
- le résidu obtenu est trituré dans l'éther éthylique afin d'éliminer l'excès de N-acétyl-β-alanine-éthylester, et obtenir environ 1,9 g de N-acétyl-β-alanyl-histamine.

**11.** Composition pharmaceutique comprenant, en association avec tout excipient approprié, un produit de couplage entre l'histamine ou l'histamine méthyl-substituée et un acide aminé de formule :

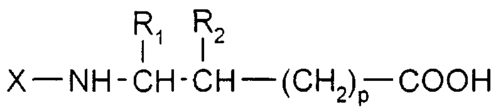

dans laquelle :

- X représente un atome d'hydrogène ou un groupe acétyle ;
- $R_1$ représente un atome d'hydrogène ou un groupe phényle ;
- $R_2$ représente un atome d'hydrogène ou un groupe méthyle ;
- p est un nombre entier compris entre 0 et 3 inclus ;

la liaison covalente avec l'histamine ou l'histamine méthyl-substituée étant une liaison peptidique entre le groupe carboxylique de l'acide aminé et le groupe amine de l'histamine ;
ledit produit étant différent de la β-alanyl-histamine.

**12.** Composition pharmaceutique selon la revendication 11, dans laquelle ledit produit est la N-acétyl-β-alanyl-histamine.

**13.** Utilisation d'un produit de couplage entre l'histamine ou l'histamine méthyl-substituée et un acide aminé selon l'une des revendications 1 à 3 pour la préparation d'un médicament destiné à soigner la cataracte.

**14.** Utilisation selon la revendication 13, pour la préparation d'un médicament destiné à soigner la cataracte sénile.

**15.** Utilisation selon la revendication 13 ou 14, dans laquelle ledit médicament est une formulation oculaire.

**16.** Utilisation selon l'une des revendications 13 à 15, dans laquelle ledit produit est la N-acétyl-β-alanyl-histamine.

**17.** Utilisation d'un produit de couplage entre l'histamine ou l'histamine méthyl-substituée et un acide aminé selon l'une des revendications 1 à 3, pour la préparation d'un médicament destiné à soigner l'athérosclérose.

**18.** Utilisation cosmétique d'un produit de couplage entre l'histamine ou l'histamine méthyl-substituée et un acide aminé selon l'une des revendications 1 à 3 comme agent anti-vieillissement cutané.

## Patentansprüche

**1.** Kupplungsprodukt zwischen Histamin oder Methyl-substituiertem Histamin und einer Aminosäure mit der Formel

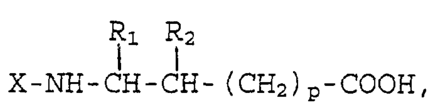

worin

- X ein Wasserstoffatom oder eine Acetylgruppe,
- $R_1$ ein Wasserstoffatom oder eine Phenylgruppe,
- $R_2$ ein Wasserstoffatom oder eine Methylgruppe und
- p eine ganze Zahl von 0 bis 3 bedeutet,

wobei die kovalente Bindung mit Histamin oder Methyl-substituiertem Histamin eine Peptidbindung zwischen der Carboxylgruppe der Aminosäure und der Aminogruppe des Histamins ist und
das Produkt sich von β-Alanylhistamin oder 6-Aminocaproyl-histamin unterscheidet.

**2.** Produkt nach Anspruch 1, in welchem die Aminosäure aus γ-Aminobuttersäure, β-Aminoisobuttersäure, 5-Aminovaleriansäure, 3-Phenyl-3-aminopropionsäure, N-Acetyl-β-alanin, N-Acetyl-3-phenyl-3-aminopropionsäure oder DL-β-Aminobuttersäure ausgewählt ist.

**3.** Produkt nach Anspruch 1 oder 2, das N-Acetyl-β-alanylhistamin ist.

**4.** Chemisches Verfahren zur Herstellung des Produkts nach einem der Ansprüche 1 bis 3 ausgehend von der Aminosäure und Histamin oder Methyl-substituiertem Histamin durch die Stufen

- N-schützen der Aminosäure durch eine X-Gruppe,

- O-aktivieren der Aminosäure durch Veresterung der Carboxylfunktion dieser Aminosäure durch Umsetzung mit einer Verbindung, die aus der Gruppe ausgewählt ist, die aus Cyanomethylalkohol, o-Nitrophenol, 2,4,5-Trichlorphenol, p-Nitrophenol, 2,4-Dinitrophenol, Pentachlorphenol, Pentafluorphenol, N-Hydroxyphthalimid, N-Hydroxybernsteinsäureimid, 1-Hydroxypiperidin und 5-Chlor-8-hydroxychinolin besteht,

- Kuppeln der N-geschützten und O-aktivierten Aminosäure mit Histamin oder Methyl-substituiertem Histamin und

- gegebenenfalls Eliminieren von X entsprechend dem verwendeten Produkt.

**5.** Enzymatisches Verfahren zur Herstellung des Produkts nach einem der Ansprüche 1 bis 3, das darin besteht, die Aminosäure mit Histamin oder Methyl-substituiertem Histamin in Gegenwart eines enzymatischen Katalysators vom Typ Hydrolase zu kuppeln.

**6.** Verfahren nach Anspruch 5, in welchem der enzymatische Katalysator eine Hydrolase ist, welche aus der Gruppe ausgewählt ist, die aus Lipasen, die aus Mikroorganismen gewonnen wurden oder tierischen Ursprungs sind, und Proteasen besteht.

**7.** Verfahren nach Anspruch 5 oder 6, welches die Stufen

- N-schützen der Aminosäure durch eine X-Gruppe,

- O-aktivieren der Aminosäure durch eine Y-Gruppe,

- Kuppeln der N-geschützten und O-aktivierten Aminosäure mit Histamin oder Methyl-substituiertem Histamin in Gegenwart des Katalysators und

- Eliminieren der X-Gruppe entprechend dem verwendeten Produkt

umfasst.

**8.** Verfahren nach Anspruch 7, in welchem die Aminosäure durch Veresterung der Carboxylfunktion dieser Aminosäure O-aktiviert wird.

**9.** Verfahren nach Anspruch 8, in welchem die Veresterungsstufe für die O-Aktivierung der Aminosäure durch Umsetzung mit einer Verbindung durchgeführt wird, die aus der Gruppe ausgewählt ist, die aus aliphatischen oder aromatischen Alkoholen besteht, wobei die tertiären Alkohole ausgeschlossen sind, und welche insbesondere Ethanol, Halogen-alkylalkohole wie 2,2,2-Trichlorethanol, Phenol, Cyanomethylalkohol, o-Nitrophenol, 2,4,5-Trichlorphenol, p-Nitrophenol, 2,4-Dinitrophenol, Pentachlorphenol, Pentafluorphenol, N-Hydroxyphthalimid, N-Hydroxybernsteinsäureimid, 1-Hydroxypiperidin und 5-Chlor-8-hydroxychinolin umfasst.

**10.** Verfahren nach einem der Ansprüche 5 bis 9, in welchem das Produkt N-Acetyl-β-alanylhistamin ist, das durch die Stufen

- Lösen von 2,38 g (15 mmol) N-Acetyl-β-alaninethylester und 1,11 g (10 mmol) Histamin in 40 ml eines Gemischs aus tert.-Amylalkohol/Heptan (25 : 75),
- Zugeben von 0,5 g Amano-P-Lipase (Pseudomonas-Spezies) und Erwärmen der Suspension bei 40 °C unter Rühren bis zum vollständigen Verschwinden des Histamins,
- Zurückgewinnen der Lipase durch Filtration,
- Verdampfen des Lösungsmittels von *tert*.-Amylalkohol/ Heptan unter Vakuum, wobei sich eine ölige Verbindung ergibt, und
- Verreiben des erhaltenen Rückstandes in Ethylether, um den Überschuß an N-Acetyl-β-alaninethylester zu entfernen und etwa 1,9 g N-Acetyl-β-alanylhistamin zu gewinnen,

hergestellt wird.

**11.** Pharmazeutische Zusammensetzung, die zusammen mit einem geeigneten Arzneimittelträger ein Kupplungsprodukt zwischen Histamin oder Methyl-substituiertem Histamin und einer Aminosäure mit der Formel

$$X\text{-}NH\text{-}\overset{\displaystyle R_1}{\overset{|}{C}}H\text{-}\overset{\displaystyle R_2}{\overset{|}{C}}H\text{-}(CH_2)_p\text{-}COOH,$$

umfasst, worin

- X ein Wasserstoffatom oder eine Acetylgruppe,
- $R_1$ ein Wasserstoffatom oder eine Phenylgruppe,
- $R_2$ ein Wasserstoffatom oder eine Methylgruppe und
- p eine ganze Zahl von 0 bis 3 bedeutet,

wobei die kovalente Bindung mit Histamin oder Methyl-substituiertem Histamin eine Peptidbindung zwischen der Carboxylgruppe der Aminosäure und der Aminogruppe des Histamins ist und
das Produkt sich von β-Alanylhistamin unterscheidet.

**12.** Pharmazeutische Zusammensetzung nach Anspruch 11, in welcher das Produkt N-Acetyl-β-alanylhistamin ist.

**13.** Verwendung eines Kupplungsprodukts zwischen Histamin oder Methyl-substituiertem Histamin und einer Aminosäure nach einem der Ansprüche 1 bis 3 für die Herstellung eines Arzneimittels zur Behandlung des grauen Stars.

**14.** Verwendung nach Anspruch 13 für die Herstellung eines Arzneimittels zur Behandlung des Altersstars.

**15.** Verwendung nach Anspruch 13 oder 14, bei welcher das Arzneimittel eine Augen-Formulierung ist.

**16.** Verwendung nach einem der Ansprüche 13 bis 15, bei welcher das Produkt N-Acetyl-β-alanylhistamin ist.

**17.** Verwendung eines Kupplungsprodukts zwischen Histamin oder Methyl-substituiertem Histamin und einer Aminosäure nach einem der Ansprüche 1 bis 3 für die Herstellung eines Arzneimittels zur Behandlung der Arteriosklerose.

**18.** Kosmetische Verwendung eines Kupplungsprodukts zwischen Histamin oder Methyl-substituiertem Histamin und einer Aminosäure nach einem der Ansprüche 1 bis 3 als Antihautalterungsmittel.

**Claims**

1. A coupling product between histamine or methyl-substituted histamine and an amino acid of the formula :

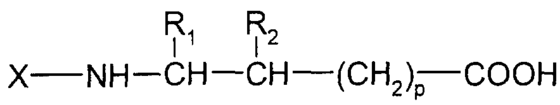

in which :

- X represents a hydrogen atom or an acetyl group ;
- $R_1$ represents a hydrogen atom or a phenyl group ;
- $R_2$ represents a hydrogen atom or a methyl group ;
- p is an integer of between 0 and 3 included ;

the covalent bond with histamine or methyl-substituted histamine being a peptide bond between the carboxylic group of the amino acid and the amine group of histamine ;
said product being different from β-alanyl-histamine or 6-aminocaproyl-histamine.

2. The product according to claim 1 wherein said amino acid is selected from γ-aminobutyric acid, β-aminoisobutyric acid, 5-aminovaleric acid, 3-phenyl-3-aminopropionic acid, N-acetyl-β-alanine, N-acetyl-3-phenyl-3-aminopriopionic acid and DL-β-aminobutyric acid.

3. The product according to claim 1 or 2 which is N-acetyl-β-alanylhistamine.

4. A chemical process for preparing the product according to one of claims 1 to 3, starting from said amino acid and histamine or methyl-substituted histamine, comprising the following steps of :

- N-protecting the amino acid by a group X ;
- O-activating the amino acid by esterification of the carboxylic function of said amino acid by reaction with a compound selected from the group consisting of cyanomethyl alcohol, o-nitrophenol, 2,4,5-trichlorophenol, p-nitrophenol, 2,4-dinitrophenol, pentachlorophenol, pentafluorophenol, N-hydroxy-phtalimide, N-hydroxysuccinimide, 1-hydroxypiperidine and 5-chloro-8-hydroxy-quinoline ;
- coupling said N-protected and O-activated amino acid with histamine or methyl-substituted histamine ; and
- eliminating or not the group X depending on the product used.

5. An enzymatic process for preparing the product according to one of claims 1 to 3 which consists in coupling the amino acid with histamine or methyl-substituted histamine in the presence of an enzymatic catalyst of the hydrolase type.

6. The process according to claim 5 wherein said enzymatic catalyst is a hydrolase selected from the group consisting of lipases extracted from micro-organisms or of animal origin, and proteases.

7. The process according to claim 5 or 6 comprising the following steps of:

- N-protecting the amino acid by a group X;
- O-activating the amino acid by a group Y ;
- coupling the N-protected and O-activated amino acid with histamine or methyl-substituted histamine in the presence of said catalyst; and
- eliminating the group X depending on the product used.

8. The process according to claim 7 wherein the amino acid is O-activated by esterification of the carboxylic function of said amino acid.

9. The process according to claim 8 wherein the esterification step of O-activating the amino acid is carried out by reaction with a compound selected from the group consisting of aliphatic and aromatic alcohols to the exclusion

of tertiary alcohols, comprising in particular ethanol, halo-alkylalcohols such as 2,2,2-trichloroethanol, phenol, cyanomethyl alcohol, o-nitrophenol, 2,4,5-trichlorophenol, p-nitrophenol, 2,4-dinitrophenol, pentachlorophenol, pentafluorophenol, N-hydroxyphtalimide, N-hydroxysuccinimide, 1-hydroxypiperidine and 5-chloro-8-hydroxyquinoline.

**10.** The process according to any one of claims 5 to 9 wherein said product is N-acetyl-β-alanyl-histamine obtained by the following steps:

- 2.38 g (15 mmoles) of N-acetyl-β-alanine ethyl ester and 1.11 g (10 mmoles) of histamine are dissolved in 40 ml of a tert-amylalcohol-heptane mixture (25:75) ;
- 0.5 g of lipase Amano P (Pseudomonas sp.) is added and the suspension is heated at 40°C under stirring until the histamine has completely disappeared ;
- the lipase is recovered by filtration ;
- the tert-amylalcohol-heptane solvent is evaporated under vacuum to give an oily compound ; and
- the residu obtained is triturated in ethyl ether to eliminate the N-acetyl-β-alanyl ethyl ester in excess and to obtain ca. 1.9 g of N-acetyl-β-alanyl-histamine.

**11.** A pharmaceutical composition comprising, in combination with any suitable excipient, a coupling product between histamine or methyl-substituted histamine and an amino acid of the formula :

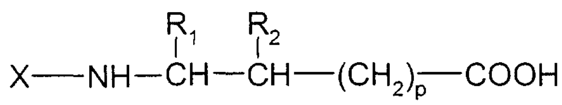

in which :

- X represents a hydrogen atom or an acetyl group ;
- $R_1$ represents a hydrogen atom or a phenyl group ;
- $R_2$ represents a hydrogen atom or a methyl group ;
- p is an integer of between 0 and 3 included ;

the covalent bond with histamine or methyl-substituted histamine being a peptide bond between the carboxylic group of the amino acid and the amine group of histamine ;
said product being different from β-alanyl-histamine.

**12.** The pharmaceutical composition according to claim 11 wherein said product is γ-aminobutyric acid, β-aminoisobutyric acid, 5-aminovaleric acid, 3-phenyl-3-aminopropionic acid, N-acetyl-β-alanine, N-acetyl-3-phenyl-3-aminopriopionic acid and DL-β-aminobutyric acid.

**13.** Use of a coupling product between histamine or methyl-substituted histamine and an amino acid according to one of claims 1 to 3 for the preparation of a medicament intended to treat cataract.

**14.** Use according to claim 13 for the preparation of a medicament intended to treat senile cataract.

**15.** Use according to claim 13 to 14 wherein said medicament is an ocular formulation.

**16.** Use according to one of claims 13 to 15 wherein said product is γ-aminobutyric acid, β-aminoisobutyric acid, 5-aminovaleric acid, 3-phenyl-3-aminopropionic acid, N-acetyl-β-alanine, N-acetyl-3-phenyl-3-aminopriopionic acid and DL-β-aminobutyric acid..

**17.** Use of a coupling product between histamine or methyl-substituted histamine and an amino acid according to one of claims 1 to 3 for the preparation of a medicament intended to treat atherosclerosis.

**18.** Cosmetic use of coupling product between histamine or methyl-substituted histamine and an amino acid according to one of claims 1 to 3 as an agent against cutaneous ageing.